(19) Europäisches Patentamt
European Patent Office
Office européen des brevets

(11) Veröffentlichungsnummer: **0 301 400 A2**

(12) # EUROPÄISCHE PATENTANMELDUNG

(21) Anmeldenummer: **88111740.2**

(51) Int. Cl.4: **C07H 15/22 , A61K 31/70**

(22) Anmeldetag: **21.07.88**

(30) Priorität: **31.07.87 CH 2938/87**

(43) Veröffentlichungstag der Anmeldung:
**01.02.89 Patentblatt 89/05**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Wessel, Hans Peter, Dr.**
**Im Bachacker 7**
**D-7843 Heitersheim(DE)**

(74) Vertreter: **Grossner, Lutz, Dr. et al**
**Grenzacher Strasse 124 Postfach 3255**
**CH-4002 Basel(CH)**

(54) **Oligosaccharidderivate, ihre Herstellung und Verwendung.**

(57) Durch Sulfatierung von Trestatinen erhält man neue Verbindungen mit anti-proliferativer und Mucosa-protektiver Wirkung.

EP 0 301 400 A2

## Oligosaccharidderivate, ihre Herstellung und Verwendung

Die vorliegende Erfindung betrifft neue sulfatierte Oligosaccharide der Formel

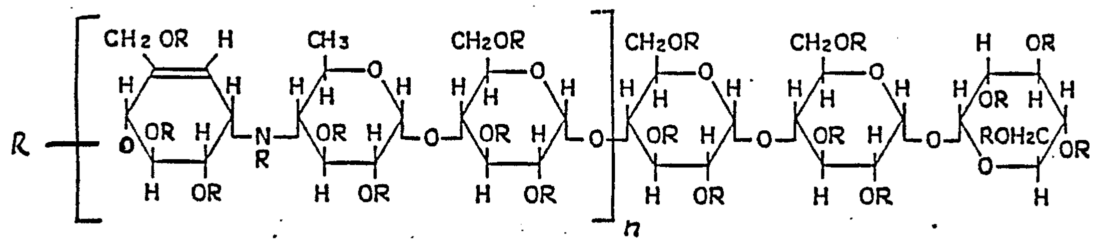

I

worin n eine ganze Zahl von 1-3; R Wasserstoff oder ein Rest $-SO_3M$ und ein M Kation ist; und wobei der Sulfatierungsgrad mindestens 1 ist.

Die Erfindung betrifft weiterhin ein Verfahren zur Herstellung der Verbindungen der Formel I, ihre Verwendung als Heilmittel bzw. als Wirkstoffe zur Herstellung von pharmazeutischen Präparaten und pharmazeutische Präparate auf der Basis von Verbindungen der Formel I.

Als Kation M kommen alle physiologisch verträglichen Kationen in Betracht, z.B. Alkalimetallkationen wie $Na^+$ und $K^+$; Ammoniumionen und substituierte Ammoniumionen, die sich von tertiären Aminen wie Triäthylamin, oder Pyridin oder Imidazol ableiten; oder quaternäre Ammoniumionen wie Dodecyltrimethylammonium, Aethylpyridinium und Benzethonium; sowie Erdalkalimetallkationen wie $Ca^{++}$. Bevorzugt sind Verbindungen, in denen M $Na^+$ ist.

Als Sulfatierungsgrad wird die Anzahl von $-SO_3M$-Resten pro Monosaccharideinheit bezeichnet, die im Molekül im Mittel vorhanden sind. Der Sulfatierungsgrad 1 liegt somit z.B. dann vor, wenn eine Verbindung der Formel I, in der n = 1 ist, 6 $-SO_3M$-Reste im Molekül enthält. Vorzugsweise beträgt der Sulfatierungsgrad in den Verbindungen der Formel I 2-3, insbesondere ca. 2,4.

Von den Verbindungen der Formel I sind diejenigen von besonderem Interesse, in denen n = 2 ist.

Die Verbindungen der Formel I können erfindungsgemäss dadurch hergestellt werden, dass man eine der Formel I entsprechende Verbindung, in der alle Reste R Wasserstoff sind, mit einem Sulfatierungsmittel behandelt.

Die Ausgangsmaterialien, d.h. den Verbindungen der Formel I entsprechende, nicht sulfatierte Verbindungen sind als Trestatin A, B und C bekanntgeworden (vgl. die europäische Patentschrift Nr. 3616).

Die Sulfatierung von Trestatin A, B oder C gemäss der vorliegenden Erfindung kann mittels Methoden, die für die Sulfatierung von Hydroxygruppen an sich bekannt sind, vorgenommen werden.

Beispiele von Sulfatierungsmitteln, die für die Herstellung der Verbindungen der Formel I angewandt werden können, sind $SO_3$•Komplexe wie $SO_3$•Pyridin, $SO_3$•Trimethylamin, $SO_3$•Dioxan und $SO_3$•Dimethylformamid. Weitere Beispiele von Sulfatierungsmitteln sind Chlorsulfonsäure, Gemische von Chlorsulfonsäure und Schwefelsäure; und Piperidin-N-Sulfat.

Als Ausgangsmaterial für die Sulfatierung kann ein einheitliches Trestatin (Trestatin A, B oder C) oder ein Gemisch von Trestatinen eingesetzt werden. Die Umsetzung erfolgt zweckmässig in einem geeigneten Lösungsmitel, insbesondere einem polaren Lösungsmittel, z.B. Dimethylformamid, Dimethylsulfoxid oder Hexamethylphosphortriamid. Die Reaktion kann bei Raumtemperatur oder erhöhter Temperatur, z.B. bei 20°C-70°C durchgeführt werden, wobei durch Variation der Reaktionsdauer und -temperatur der Sulfatierungsgrad beeinflusst werden kann. Der jeweils erreichte Sulfatierungsgrad kann durch HPLC abgeschätzt werden. Die Aufarbeitung des Reaktionsgemisches bzw. die Isolierung des Reaktionsprodukts der Formel I kann nach an sich bekannten Methoden aus dem Reaktionsgemisch erfolgen, z.B. durch Gelfiltration oder Ultrafiltration.

Die Verbindungen der Formel I hemmen die Migration und Proliferation von Zellen der vaskulären glatten Muskulatur und verhüten proliferative arteriosklerotische Läsionen, ohne einen Einfluss auf das Wachstum von Endothelzellen aufzuweisen. Ihre blutgerinnungshemmende Aktivität ist geringer als die von Heparin. Die Verbindungen der Formel I können daher zur Prophylaxe der Arteriosklerose, insbesondere nach Bypass-Operationen oder Angioplastie, sowie zur Behandlung von Patienten mit progressiver Arteriosklerose verwendet werden.

Die Verbindungen der Formel I zeigen weiterhin eine Mucosa-protektive Wirkung und können daher zur Therapie und Prophylaxe von Magengeschwüren verwendet werden.

Die blutgerinnungshemmende Wirkung wurde wie folgt ermittelt:

aPTT (activated partial thromboplastin time)-Test (vgl. Walenga et al., CRC Critical Reviews in Laboratory Sciences 22 (4) 361-389 (1986)): 100 µl citriertes menschliches Plasma, das verschiedene Konzentrationen der Versuchsverbindung enthält, wird mit 100 µl Activated Thrombofax (Ortho Diagnostics, Raritan, N.J., U.S.A.) 8 Minuten bei 37 °C gemischt. Dann werden 100 µl vorgewärmte 25 mM Calciumchloridlösung zugesetzt und die Gerinnungszeit wird im Fibrometer Coagulation Timer (Becton, Dickinson Basel) gemessen.

anti-Xa Clotting Assay: 25 µl citriertes Plasma mit verschiedenen Konzentrationen der Versuchsverbindung werden mit 75 µl 1:100 mit 0,63% Citratpuffer (pH 7,3), der 41 mM Imidazol, 82 mM NaCl und 0,1% Albumin enthält, verdünntem Factor Xa (Diagnostic Reagents, Thame, Oxon, Grossbritannien) gemischt. Nach 2 Minuten Erwärmen auf 37 °C werden 200 µl eines 1:1-Gemisches von Factor X Deficient Plasma (Diagnostic Reagents) und Platelet Substitute (Diagnostic Reagents) zugesetzt und das Gemisch 20 Sekunden bei 37 °C inkubiert. Nach Zusatz von 100 µl vorgewärmter 50 mM Calciumchloridlösung wird die Gerinnungszeit im Fibrometer gemessen.

Die Aktivität der Versuchsverbindung wird als $IC_{50}$ angegeben, die die Konzentration [µg/ml] angibt, die zu einer Gerinnungszeit führt, die das Doppelte des Kontrollwerts beträgt.

Hemmung von Thrombin oder Faktor Xa im Chromogenic Substrate Assay (Teien et al., Thrombosis Research 10, 399-410 (1977)): Die Bestimmung erfolgte im Cobas-Bio Centrifugal Automatic Spectrophotometer. Die verwendete Pufferlösung bestand aus 50 mM Tris-Buffer, 180 mM NaCl, 7,5 mM $EDTA\text{-}Na_2$, 1% PEG 6000 und 0,02% Tween-80, pH 8,4. Die Testlösung bestand aus 50 µl Puffer, 30 µl Antithrombin III (1 U/ml, Kabi Diagnostica) und 20 µl Plasma, das verschiedene Konzentrationen der Versuchverbindungen enthielt. Im Analysenautomat wurden 30 µl Probenlösung und 20 µl Wasser mit 180 µl Thrombin (1 U/ml, Thrombin Reagent Roche Basel) in die Testküvette gegeben. Nach 240 Sekunden Inkubation bei 37 °C wurden 60 µl S-2238 (H-D-Phe-Pip-Arg-NH.pNA, Kabi Diagnostica, Möndal, Schweden, 0,75 mM in Wasser) und 20 µl Wasser zugesetzt. Die Freisetzung von pNA (p-Nitroanilin) wurde während 60 Sekunden bei 405 nm in 10-Sekunden-Intervallen im Vergleich zu Wasser als Blindwert verfolgt. Die Hemmwirkung wird als $IC_{50}$ angegeben, was die Konzentration [µg/ml] angibt, bei der die amidolytische Aktivität von Thrombin im Vergleich zum Plasma-Kontrollwert um 50% vermindert wird.

In gleicher Weise wurde die Hemmung von Faktor Xa gemessen, wobei eine Lösung von Faktor Xa (2,8 nkat/ml und 2 mM S-2222 (Bz-CO-Ile-Glu-Arg-NH.pNA, Kabi Diagnostica) in Wasser anstelle von Thrombin bzw. S-2238 verwendet wurde.

Die Resultate, die in den oben beschriebenen Versuchsanordnungen mit der Verindung von Beispiel 1 und Heparin erhalten wurde, sind nachstehend angeführt:

| | Gerinnungshemmung $IC_{50}$ (µg/ml) | | Chromogenic Substrate (amidolytische Aktivität) $IC_{50}$ (µg/ml) | |
|---|---|---|---|---|
| | aPTT | Xa | IIa | Xa |
| Heparin (Standard) | 1,2 | 0,6 | 1,9 | 2,7 |
| Verbindung von Beispiel 1 | 7 | >30 | >1000 | 550 |

Die anti-proliferative Wirksamkeit wurde wie folgt ermittelt:

Zellen verschiedenen Ursprungs (Swiss 3T3 Mäusefibroblastoide Zellen, HSMC: menschliche glatte Muskelzellen und Endothelzellen, beide aus menschlichen Nabelschnurvenen isoliert) wurden in RPMI 1640 Medium enthaltend 10% Kälberserum (Swiss 3T3 Zellen), 15% foetales Kälberserum (HSMC) oder 15% foetales Kälberserum plus 300 µg/ml endothelial cell growth supplement (Endothelzellen) bei 37 °C in wassergesättigter Luft/5% $CO_2$ kultiviert. Konfluente Kulturen wurden dann einmal mit HBSS gewaschen, mit 0,05% Trypsin/0,02% EDTA abgelöst und durch Zentrifugieren gesammelt. Die Zellen wurden auf Zellkulturplatten mit einer Dichte von 5000 Zellen/Kultur aufgebracht und die Versuchsverbindung eine Stunde später zugesetzt. Die Zellen wurden dann bei 37 °C in Luft/5% $CO_2$ inkubiert und am 4. Tag (Swiss 3T3) bzw. am 7. Tag (HSMC und Endothelzellen) gesammelt. Zur Bestimmung der Anzahl Zellen wurden diese einmal mit HBSS gewaschen und festhängende Zellen zur Ablösung 10 Minuten bei 37 °C mit 0,5 ml Trypsin/EDTA-Lösung inkubiert. Zur Fixierung der Zellen wurde zu jeder Kultur 0,5 ml Formol-Lösung (0,5% Paraformaldehyd, 0,145 M NaCl, 1,3 mM EDTA) gegeben. Zur Vermeidung des Klumpens wurden die Zellsuspensionen 20 mal auf und ab pipettiert. Die Zellzahlen wurden mit einem Coulter-Counter bestimmt. Die Hemmwirkung durch die Versuchsverbindungen wurde wie folgt bestimmt:

$$\% \text{ Hemmung} = \left[ 1 - \frac{\text{Nettoproliferation mit Versuchssubstanz}}{\text{Nettoproliferation in der Kontrollreihe}} \right] \times 100$$

wobei die Nettoproliferation die Anzahl Zellen am Versuchsende minus die Anzahl Zellen am Tag 1 (=24 Stunden nach Beimpfen) ist. Der $IC_{50}$-Wert wurde aus der Kurve % Hemmung gegen log Inhibitorkonzentration extrapoliert.

Die Resultate dieser Versuche sind nachstehend aufgeführt:

| | $IC_{50}$ [µg/ml] | |
|---|---|---|
| | Swiss 3T3 | HSCM |
| Heparin (Standard) | 35 | 45 |
| Verbindung von Beispiel 1 | 30 | 20 |

Zur Bestimmung der muscosa-protektiven Eigenschaft kann die nachstehend beschriebene Versuchsanordnung verwendet werden:

Orale Verabreichung von absolutem Aethanol an männliche Ratten in einer Dosis von 1 ml pro Ratte führt innerhalb 1 Stunde zu blutigen Läsionen der Magenschleimhaut. Verschiedene Dosen der zu prüfenden Substanzen (suspendiert in 0,125% Carboxymethylcellulose) oder das Vehikel allein (Kontrolle) werden den Ratten oral (1 ml pro Ratte) 30 Minuten vor der Behandlung mit Aethanol verabreicht. Eine Stunde nach der Verabreichung des Aethanols tötet man die Tiere, untersucht deren Mägen auf das Vorhandensein von Läsionen und ermittelt die Anzahl und die gesamte Ausdehnung solcher Läsionen.

Bei Verabreichung von 30 mg/kg der Verbindung von Beispiel 1 wurde die Anzahl der Läsionen im Vergleich zur Kontrollgruppe um 28% reduziert.

In einer akuten Toxizitätsbestimmung an der Maus war die Verbindung von Beispiel 1 nach intravenöser Applikation von 500 mg/kg bzw. nach subkutaner Applikation von 4000 mg/kg noch nicht letal.

Wie eingangs erwähnt, sind Arzneimittel, enthaltend eine Verbindung der Formel I ebenfalls Gegenstand der vorliegenden Erfindung, weiterhin auch ein Verfahren zur Herstellung derartiger Arzneimittel, welches dadurch gekennzeichnet ist, dass man eine oder mehrere Verbindungen der Formel I und erwünschtenfalls einen oder mehrere andere therapeutisch wertvolle Stoffe in einer galenische Darreichungsform bringt.

Die Arzneimittel können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, verabreicht werden. Die Verabreichung erfolgt jedoch vorzugsweise parenteral, z.B. in Form von Injektionslösungen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Bei enteraler Verabreichung kann die Resorption des Wirkstoffs mit Hilfe von Liposomen erhöht werden.

Die Dosierung des Wirkstoffes kann innerhalb weiter Grenzen variieren und ist natürlich in jedem einzelnen Fall den individuellen Gegebenheiten anzupassen. Im allgemeinen dürfte bei parenteraler Verabreichung eine Dosis von etwa 0,1 bis 100 mg/kg, vorzugsweise von etwa 1,5 bis 15 mg/kg, pro Tag für den Erwachsenen angemessen sein, wobei aber die soeben angegebene obere Grenze auch überschritten werden kann, wenn sich dies als angezeigt erweisen sollte.

## Beispiel 1

Am Hochvakuum getrocknetes Trestatin A (1,0 g, 697 µMol) wurde in Dimethylformamid (30 ml) gelöst und mit $SO_3$-Pyridin-Komplex (6,8 g, 42,5 mMol) versetzt. Die Lösung trübte sich innerhalb weniger Minuten durch allmähliches Ausfallen eines bräunlichen Oels. Nach 16 Stunden Rühren wurde die überstehende Dimethylformamid-Lösung abdekantiert und das verbleibende Oel mit Methanol gewaschen. Eine analytische Menge des Oels wurde an einer Sephadex G50-Säule gelchromatographiert und der Sulfatierungsgrad NMR-spektroskopisch bestimmt (Integralvergleich der Pyridin- und Methylprotonen). Die Hauptmenge wurde mit 10%iger Natriumacetatlösung versetzt und im Vakuum eingedampft, nochmals mit Wasser gelöst und eingedampft, bis sämtliches Pyridin entfernt war. Zur Reinigung des entstandenen Trestatin A-Sulfat-Natriumsalzes wurde an Sephadex G50 chromatographiert. Aus der Elementaranalyse (18,86% S) ergibt sich, dass das Produkt einen Sulfatierungsgrad n = 2,3 aufweist. Das NMR-Spektrum ist in Figur 1 dargestellt (interner Standard: Natrium-2,2,3,3-tetradeutero-3-trimethylsilylpropionat, aufgenommen mit Bruker HX-279).

## Beispiel 2

Zur Herstellung einer Injektionslösung werden 5 mg der Verbindung von Beispiel 1 und 9 mg Natriumchlorid in Wasser ad 1 ml gelöst. Die Lösung wird mit Ascorbinsäure (0,5 mg/ml) und Benzylalkohol (0,1 mg/ml) versetzt und dann sterilfiltriert.

**Ansprüche**

1. Verbindungen der Formel

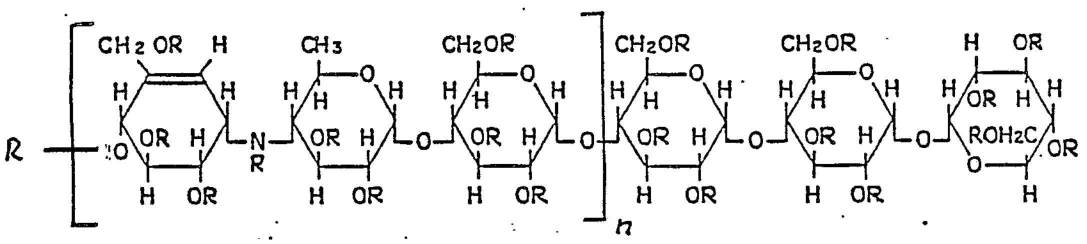

I

worin n eine ganze Zahl von 1-3; R Wasserstoff oder ein rest $-SO_3M$ und ein M kation ist; und wobei der Sulfatierungsgrad mindestens 1 ist.

2. Verbindungen gemäss Anspruch 1, in denen n = 2 ist.

3. Verbindungen gemäss den Ansprüchen 1 oder 2, in denen der Sulfatierungsgrad 2-3 ist.

4. Die Verbindungen gemäss den Ansprüchen 1-3 zur Anwendung als Heilmittel, insbesondere zur Therapie und Prophylaxe der Arteriosklerose.

5. Die Verbindungen gemäss den Ansprüchen 1-3 zur Verwendung als Wirkstoffe bei der Herstellung von pharmazeutischen Präparaten, insbesondere zur Therapie und Prophylaxe der Arteriosklerose.

6. Pharmazeutische Präparate, enthaltend eine Verbindung gemäss den Ansprüchen 1-3 und übliche pharmazeutische Trägerstoffe.

7. Verfahren zur Herstellung von Verbindungen der Formel I, dadurch gekennzeichnet, dass man eine der Formel I entsprechende Verbindung, in der alle Reste R Wasserstoff sind, mit einem Sulfatierungsmittel behandelt.

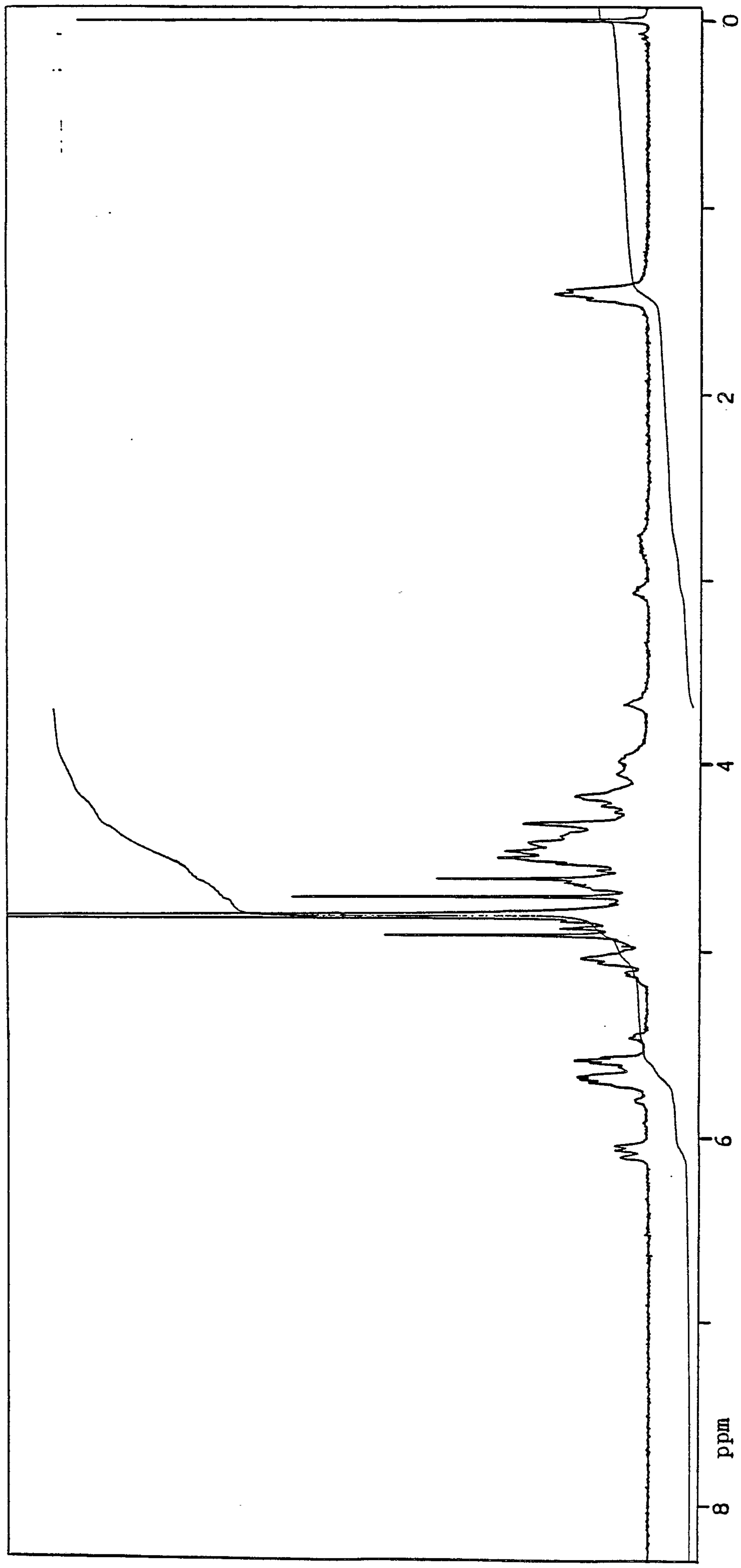


Figur 1